Europäisches Patentamt

**European Patent Office**

**Office européen des brevets**

(19)

(11) Publication number: **0 014 324**
**B1**

(12) # EUROPEAN PATENT SPECIFICATION

(45) Date of publication of patent specification: **13.07.83**

(51) Int. Cl.³: **A 61 B 5/12**

(21) Application number: **80100113.2**

(22) Date of filing: **11.01.80**

(54) **Device for measuring the acoustical ear impedance.**

(30) Priority: **12.01.79 IT 6705879**

(43) Date of publication of application:
**20.08.80 Bulletin 80/17**

(45) Publication of the grant of the patent:
**13.07.83 Bulletin 83/28**

(84) Designated Contracting States:
**DE FR GB NL**

(56) References cited:
**DD - A - 80 965**
**US - A - 3 294 193**
**US - A - 3 949 735**
**US - A - 4 009 707**
**US - A - 4 057 051**
**US - A - 4 079 198**

(73) Proprietor: **CSELT Centro Studi e Laboratori Telecomunicazioni S.p.A.**
**Via Guglielmo Reiss Romoli, 274**
**I-10148 Turin (IT)**

(72) Inventor: **Canavesio, Franco**
**Via Donizetti 17**
**Torino (IT)**
Inventor: **Ceruti, Rodolfo**
**Via Tenivelli 21**
**Torino (IT)**

(74) Representative: **Riederer Freiherr von Paar zu Schönau, Anton et al,**
**Freyung 615 Postfach 2664**
**D-8300 Landshut (DE)**

Courier Press, Leamington Spa, England

**0 014 324**

## Device for measuring the acoustical impedance of the ear

The present invention relates to a device for measuring the acoustical impedance of the ear upon evaluating the transfer function of an open loop comprising a probe to be hermetically coupled to the ear or to calibrating standard cavities, respectively, and, coupled to the probe via small tubes, an exciting electro-acoustical transducer, a microphone transducer and a static pressure generator which generates selectable values of static pressure, the device further comprising:

—an electrical signal generator supplying an exciting ac signal within the audible frequency range to the exciting transducer causing it to issue a test sound signal,

—a signal-analyzing circuit fed by the signal issued by the microphone transducer upon issuance of the test sound signal, and

—a processing unit connected to receive the output signal of the signal-analyzing circuit and calculating thereupon the acoustical impedance of the ear for the respective data.

E.g. a device for measuring the acoustical impedance of the ear by means of sine oscillations has become known (US—A 4 009 707), comprising a generator of the electrical sine signals supplied to an exciting transducer and a signal-analyzing circuit connected to a microphone-transducer and receiving the electrical wave emitted therefrom, the signal-analyzing circuit derived from the received electrical wave for a given static bias pressure value sets of amplitude samples. The sine frequency is selectable. Further, an electro-acoustical impedance meter is known (US—A 4 079 198) utilizing a bridge arrangement and an artificial comparison ear. The arrangement uses a loudspeaker, a microphone, conduits, a bias pressure and an ear plug. For the measurement, rising and falling frequencies according to a ramp are used.

Measurement over a wider frequency range requires long operating times involving a lot of inconveniences of practical kind which may limit its application particularly in the clinical field.

Thereagainst, the problem is to carry out the impedance measurement over a wider frequency range without any complications and considerable extensions of time. This problem is solved by the present invention which is characterized in that the electrical signal generator is a generator issuing wide frequency spectrum signals containing frequencies at least between 100 Hz and 4500 Hz, that the signal-analyzing circuit supplies amplitude samples of the response of the microphone transducer and that to the processing unit a memory is associated wherein the amplitude samples of the calibrating tests wherein the probe is coupled to the calibrating standard cavities and the amplitude samples of the ear test wherein the probe is coupled for the ear under test are storable for different static pressures and the different frequency values and are readable for further processing.

Preferably, the processing unit carries out the following processing operation on the amplitude samples stored in the memory:

$$Z_{xi}(\omega) = \frac{\gamma P_i}{j\pi c a_1^2} \cot \frac{\omega L_1}{c} \cdot \frac{\dfrac{S_{xi}(\omega)}{S_{1i}(\omega)}\left(\dfrac{S_{1i}(\omega)}{S_{2i}(\omega)} - 1\right)}{\left(\dfrac{a_2^2 \cot \omega L_1/c}{a_1^2 \cot \omega L_2/c} - 1\right) - \dfrac{S_{xi}(\omega)}{S_{1i}(\omega)}\left(\dfrac{a_2^2 \cot \omega L_1/c}{a_1^2 \cot \omega L_2/c} - \dfrac{S_{1i}(\omega)}{S_{2i}(\omega)}\right)}$$

wherein:

$Z_{xi}(\omega)$ = the accoustical impedance of the ear for a particular angular frequency $\omega$ and for static pressure $P_i$;

$S_{xi}(\omega)$ = ear amplitude samples for $\omega$ and $P_1$;

$S_{1i}(\omega)$ = first calibrating standard cavity amplitude samples for $\omega$ and $P_i$;

$S_{2i}(\omega)$ = second calibrating standard cavity amplitude samples for $\omega$ and $P_i$;

$a_1, L_1$ = radius and length, respectively, of the first cylindrical calibrating standard cavity;

$a_2, L_2$ = radius and length, respectively, of the second cylindrical calibrating standard cavity;

$C$ = propagation speed of sound in air; and

$\gamma$ = a fixed parameter.

These and other characteristics of the present invention will become clearer from the following description of a preferred embodiment thereof given by way of example and not in a limiting sense, taken in conjunction with the annexed drawings in which:

Fig. 1 represents a block diagram of the technique of measurement used;

Fig. 2 is a diagram relating the energizing electrical pulses to the response produced by the microphone denoted by M in Fig. 1;

Fig. 3 is the axonometric representation of a particular example of a family of curves formed by the trends of the acoustical impedance $Z_{xi}(\omega)$ in function of the frequency ($f = \omega/2\pi$) and of the static pressure $P_1$.

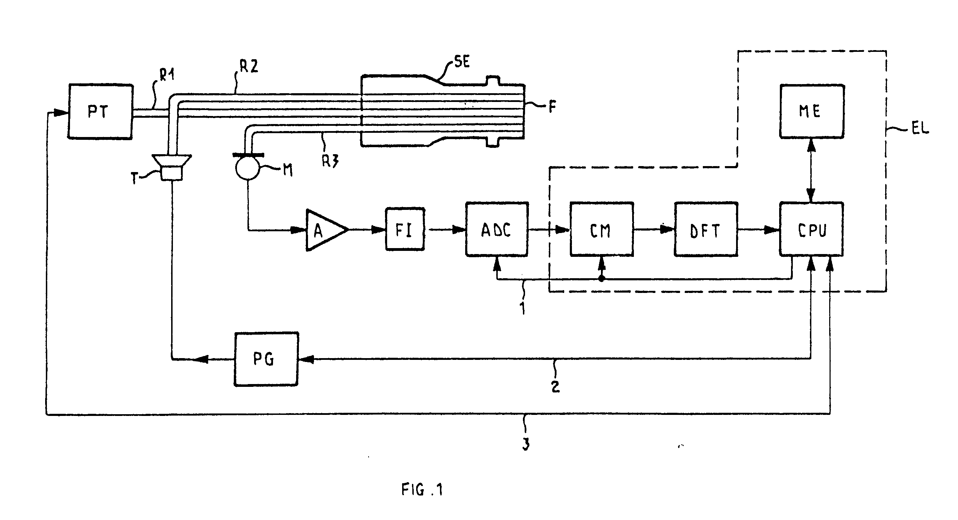

In Fig. 1 reference SE denotes a probe of known type to be hermetically applied to the external ear.

Reference T denotes an electroacoustical transducer acoustically connected through a small tube R2 to probe SE.

Reference M denotes a microphone transducer of any known type acoustically connected through a small tube R3 to probe SE.

Reference PT denotes a static pressure generator of any known type, whose output is connected to small tube R1 also inserted into probe SE.

The small tubes R1, R2, R3 are inserted into probe SE, so that they can just protrude from the open bottom F, where the gaps comprised among the small tubes and the inner walls of probe SE, are duly sealed.

Reference A denotes an amplifier of known type, able to receive at the input and duly amplify the electrical signal originated from microphone M.

Reference FI denotes a low-pass filter of known type connected to the output of A; the cutoff frequency of the filter is $f_o$.

Reference ADC denotes an analog-to-digital converter of known type, able to receive the filtered and amplified signal coming from amplifier A and filter FI and convert it into digital form.

It is now worth noticing that the presence of the filter is uncesessary when the filtering function is an inherent characteristic of the operation of transducers T or M.

In fact transducers having a frequency-limited response similar in trend to the transfer function of a low-pass filter are commercially available.

Reference CM denotes a digital device of known type able to compute the average of the digital signals received from ADC, to which it is connected at the input.

As known, a device like CM has the function of ameliorating the signal-to-noise ratio.

Reference DFT denotes a usual circuit able to calculate the Discrete Fourier Transform of the average signal it receives from CM, to which it is connected. More particularly the circuit DFT can implement the algorithm of the Fast Fourier Transform (FFT). As known, the function of a circuit like DFT is that of supplying the frequency spectrum of the pulse extracted from the microphone, therfore at the output of DFT for a sufficiently narrow exciting pulse the overall transfer function between T and M will be obtained.

Reference CPU denotes a usual processing unit connected to a memory, denoted by ME; CPU is able to receive the data supplied by DFT and to process them, according to an algorithm which will be described hereinafter, in order to calculate the acoustical impedance of the ear under test.

In additon CPU is able to synchronize the sampling acquisition and average of the pulse response by the following commands:

—a synchronizing command, sent to ADC and CM through connections, designed to time the sampling process and effectuate a coherent average operation, respectively;

—a starting command for a pulse generator, denoted by PG, transmitted through the two-way (bidirectional) connection 2, through which CPU also receives the pulse just emitted by PG with confirmation meaning;

—a command signal of the static pressure generated by PT; said signal is transmitted through connection 3.

Blocks denoted by CPU, CM, DFT and ME just examined can consist also of a single processing unit EL of any known type, provided it can operate at the speed required by the measurement, and is duly interfaced.

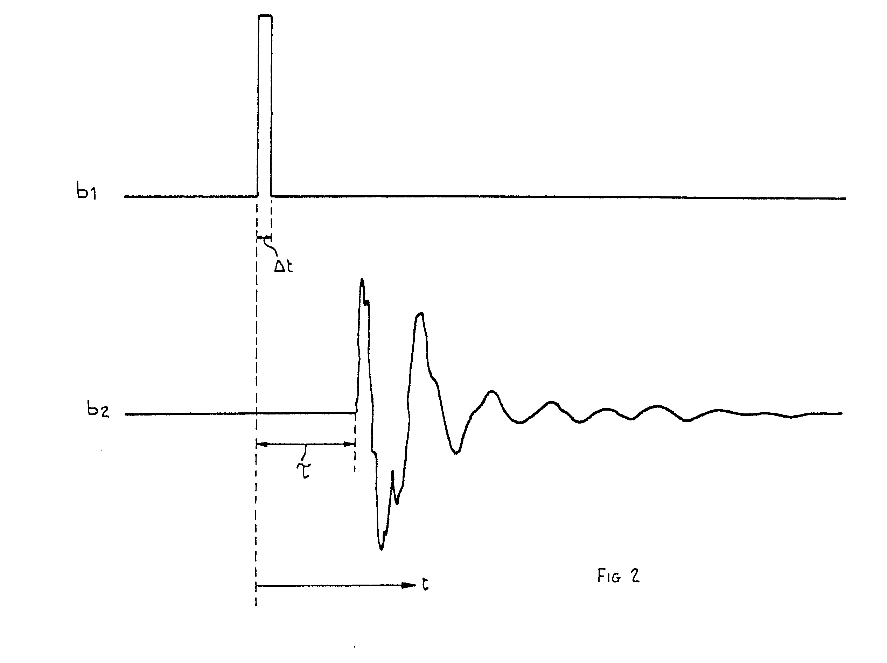

In Fig. 2, line $b_1$ represents the exciting electrical pulse, assumed to have duration $\Delta t \leq 1/2\, f_o$, originated from generator PG (Fig. 1) and sent to electroacoustical transducer T; curve $b_2$ (Fig. 2) represents the electrical signal generated by microphone M (Fig. 1) in correspondence with the pulse of line $b_1$ (Fig. 2), sent to the input of amplifier A (Fig. 1).

Reference $\tau$ (Fig. 2) denotes the delay elapsed between the beginning of the pulse represented in $b_1$ and the beginning of the response signal generated by microphone M; said delay $\tau$ is due to the propagation time of the sound through the small tubes R2, R3 and being systematic it can be easily taken into account both during the automatic effectuation of a measurement and during the following processing operation.

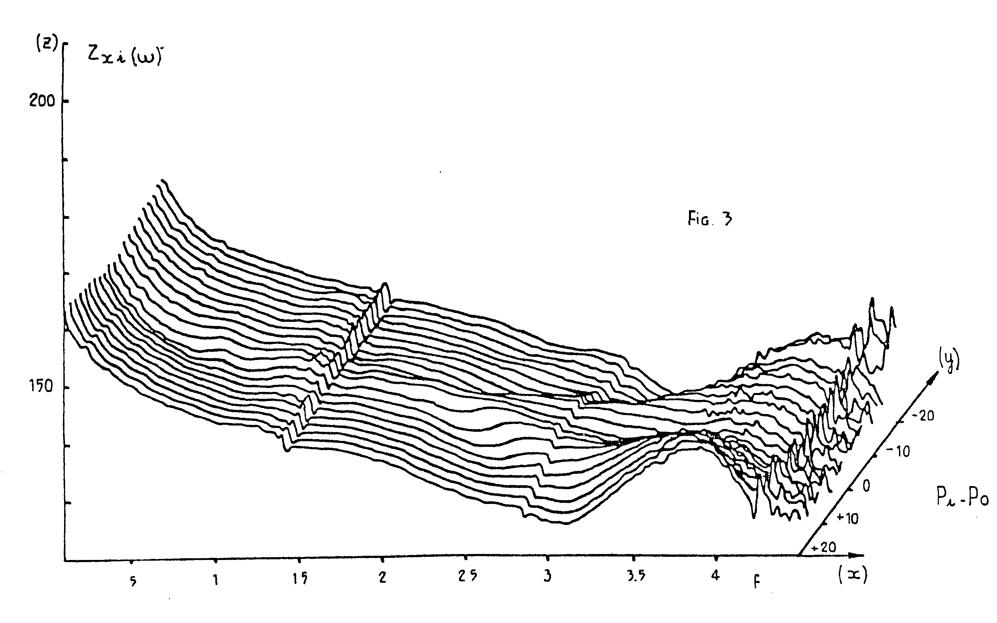

In Fig. 3 the parameter of the various curves of the represented family is static pressure $P_1$, whose difference with respect to $P_o$ is measured in water centimeters and reported on orthogonal coordinate y; the values in modulus of the acoustical impedance $Z_{xi}(\omega)$ are plotted on coordinate z and are expressed in dB referred to $1[N.sec/m^5]$; frequency values in kHz are reported on axis x.

It is worth noting that each curve of the family is obtained as a result of the processing operation deriving from the excitation produced by a pulse like the one depicted in Fig. 2.

Obviously, to increase the signal-to-noise ratio it can be convenient to produce the excitation by means of a plurality of pulses, repeated with a prefixed rhythm, so as to let the digital device (CM) of Fig. 2 effect on the same the averaging in a coherent way.

3

Now the algorithm is briefly mentioned from which the inventors have started for determining the way of measurement.

By calling $S_1(\omega)$ and $S_2(\omega)$ the transfer functions obtained by coupling the probe SE (Fig. 1) to two calibrating cylindrical cavities $C_1$ and $C_2$ (not shown in the Figure) having volume $V_1$ and $V_2$ respectively and by calling $S_x(\omega)$ the transfer function obtained by coupling the probe SE on an unknown impedance $Z_x(\omega)$, according to the literature the validity of the following expression is proved:

$$Z_x(\omega) = \frac{\gamma P_o}{j\omega V_1} \left\{ \frac{\dfrac{S_x(\omega)}{S_1(\omega)}\left[\dfrac{S_1(\omega)}{S_2(\omega)} - 1\right]}{\left[\dfrac{V_2}{V_1} - 1\right] - \dfrac{S_x(\omega)}{S_1(\omega)}\left[\dfrac{V_2}{V_1} - \dfrac{S_1(\omega)}{S_2(\omega)}\right]} \right\} \tag{1}$$

where $\gamma = c_p/c_v$ (ratio of specific heat at constant pressure $c_p$ to that at constant value $c_v$), in the air is $\gamma = 1.41$; $P_o$ = atmospheric pressure, $\omega$ = angular frequency; $j = \sqrt{-1}$.

Formula (1) can be perfectly applied to known techniques of measurement that utilize the sinusoidal condition and supplies the unknown impedance $Z_x(\omega)$ in function of known physical and geometric quantites ($P_o$, $V_1$, $V_2$, $\gamma$), of transfer functions $S_1(\omega)$ and $S_2(\omega)$ obtained once for all during the calibration of the probe SE and in function of the measure of $S_x(\omega)$.

However, expressed as above, formula (1) cannot be applied to wide spectrum exciting techniques, that are object of the present invention, as (1) does not take into due account the relative dimensions and the geometric shape of the calibration cavities $C_1$ and $C_2$ with the result of limiting the field frequency in which it operates.

The inventors have modified the expression of the formula (1) in order to take into account, with the same volume, the geometric shape of such cavities $C_1$ and $C_2$, and in case of cylindrical cavities, the values of the depth and of the radius of the section.

More particularly, considering the exact formula, known in the literature, of the acoustical impedance of a cylindrical cavity with hard walls (L. Beranek—Acoustics, McGraw-Hill, 1954, P. 128), and after calling L1 and L2 the depth of cavity $C_1$ and $C_2$ respectively, the formula (1) becomes:

$$Zxi(\omega) = \frac{\gamma \cdot P_i}{j\pi c a_1^2} \cot \frac{\omega L_1}{c} \cdot \frac{\dfrac{S_{xi}(\omega)}{S_{1i}(\omega)}\left(\dfrac{S_{1i}(\omega)}{S_{2i}(\omega)} - 1\right)}{\left(\dfrac{a_2^2 \cot \omega L_1/c}{a_1^2 \cot \omega L_2/c} - 1\right) - \dfrac{S_{xi}(\omega)}{S_{1i}(\omega)}\left(\dfrac{a_2^2 \cot \omega L_1/c}{a_1^2 \cot \omega L_2/c} - \dfrac{S_{1i}(\omega)}{S_{2i}(\omega)}\right)} \tag{2}$$

where = C velocity of sound in the air; $a_1$, $a_2$ = radiuses of the bases of the cylindrical cavities $C_1$ and $C_2$; $L_1$, $L_2$ = height of the cylinders forming cavities $C_1$ and $C_2$; $P_1$ = static pressure corresponding to 1 water centimeter.

In formula (2) the expressions $S_{xi}(\omega)$, $S_{1i}(\omega)$, $S_{2i}(\omega)$ are the transfer functions $S_x(\omega)$, $S_1(\omega)$ and $S_2(\omega)$, already examined in formula (1), this time not determined at atmospheric pressure $P_o$ but at various values of static pressure $P_1$.

Formula (2) is implemented by the scheme of Fig. 1 for the measurement of the acoustical impedance $Z_{xi}(\omega)$ of an ear by using techniques of wide spectrum excitation.

Now the way of measurement will be described and consequently the working operation of the system of Fig. 1 will be described.

The system calibration to be effected once for all and after any possible replacement of parts forming the system is carried out in the following way.

Probe SE is hermetically coupled to a first calibration cylindrical cavity $C_1$, with known dimentions and consequently with known acoustical impedance.

Then EL detects and memorizes the spectrum of the transferred pulse, that corresponds to $S_{1i}(\omega)$ under the hypothesis already assumed that, calling $f_o$ the cutoff frequency of filter FI, the pulses generated by PG have a spectrum flat enough in the measurement area, that is they have $\Delta t \leq 1/2 f_o$ width. Obviously the sampling frequency $f_c$ of converter ADC must be: $f_c \geq 2 f_o$.

4

The detection and storage of said transfer functions are repeated on the whole range of pressure values originated by PT in order to take into account also the possible variations of the electroacoustical properties of M and T in function of the static pressure. This allows the use of not too sophisticated transducers even ensuring the accuracy in the measurements.

Probe SE is hermetically coupled to a second calibration cavity, thus determining and memorizing, in a perfectly analogous way, $S_{2i}(\omega)$.

The actual measurement of the acoustical impedance $Z_{xi}(\omega)$ is performed as follows. Probe SE is hermetically coupled to the ear under test; then the transfer function $S_{xi}(\omega)$ is determined and memorized in an analogous way as done for $S_{1i}(\omega)$ and $S_{2i}(\omega)$.

Applying formula (2) the value of $Z_{xi}(\omega)$ is obtained. Of course the task of calculation of $Z_{xi}(\omega)$ in function of $S_{1i}(\omega)$, $S_{2i}(\omega)$, and $S_{xi}(\omega)$ is given to CPU (or more generically to EL) where the values of depth and of the radiuses of section of cavities $C_1$ and $c_2$ have been previously memorized.

The processing unit CPU (or EL) is programmed, in any known way, for controlling checks and computations necessary to perform the measurement.

Obviously the scheme of the measurement system depict in Fig. 1 can be varied and modified without going out of the scope of the invention.

For instance, as PT generates in any case a static pressure, it is possible to convey into a single small tube the small tubes R1 and R2, or R1 and R3, thus obtained to limit to two the small tubes introduced into probes SE with consequent simplified structure.

## Claims

1. Device for measuring the acoustical impedance of the ear upon evaluating the transfer function of an open loop comprising a probe (SE) to be hermetically coupled to the ear or to calibrating standard cavities, respectively, and, coupled to the probe via small tubes (R1, R2, R3), an exciting electro-acoustical transducer (T), a microphone transducer (M) and a static pressure generator (PT) which generates selectable values of static pressure ($P_i$), the device further comprising:
—an electrical signal generator (PG) supplying an exciting ac signal within the audible frequency range to the exciting transducer (T) causing it to issue a test sound signal,
—a signal-analyzing circuit (ADC, CM, DFT) fed by the signal issued by the microphone transducer (M) upon issuance of the test sound signal, and
—a processing unit (CPU) connected to receive the output signal of the signal-analyzing circuit (ADC, CM, DFT) and calculating thereupon the acoustical impedance of the ear for the respective data, characterized in that the electrical signal generator (PG) is a generator issuing wide frequency spectrum signals containing frequencies at least between 100 Hz and 4500 Hz, that the signal-analyzing circuit (ADC, CM, DFT) supplies amplitude samples of the response of the microphone transducer (M) and that to the processing unit (CPU) a memory (ME) is associated wherein the amplitude samples of the calibrating tests wherein the probe (SE) is coupled to the calibrating standard cavities and the amplitude samples of the ear test wherein the probe (SE) is coupled to the ear under test are storable for different static pressures ($P_1$) and the different frequency values and are readable for further processing.

2. Device according to claim 1, characterized in that the processing unit (CPU) is designed to carry out the following processing operation on the amplitude samples stored in the memory (ME):

$$Z_{xi}(\omega) = \frac{\gamma P_i}{j\pi c a_1^2} \cot \frac{\omega L_1}{c} \cdot \frac{\dfrac{S_{xi}(\omega)}{S_{1i}(\omega)}\left(\dfrac{S_{1i}(\omega)}{S_{2i}(\omega)} - 1\right)}{\left(\dfrac{a_2^2 \cot \omega L_1/c}{a_1^2 \cot \omega L_2/c} - 1\right) - \dfrac{S_{xi}(\omega)}{S_{1i}(\omega)}\left(\dfrac{a_2^2 \cot \omega L_1/c}{a_1^2 \cot \omega L_2/c} - \dfrac{S_{1i}(\omega)}{S_{2i}(\omega)}\right)}$$

wherein:
$Z_{xi}(\omega)$ = the accoustical impedance of the ear for a particular angular frequency $\omega$ and for static pressure $P_i$;
$S_{xi}(\omega)$ = ear amplitude samples for $\omega$ and $P_1$;
$S_{1i}(\omega)$ = first calibrating standard cavity amplitude samples for $\omega$ and $P_i$;
$S_{2i}(\omega)$ = second calibrating standard cavity amplitude samples for $\omega$ and $P_i$;
$a_1$, $L_1$ = radius and length, respectively, of the first cylindrical calibrating standard cavity;
$a_2$, $L_2$ = radius and length, respectively, of the second cylindrical calibrating standard cavity;
$C$ = propagation speed of sound in air; and
$\gamma$ = a fixed parameter.

3. Device according to claim 2, characterized in that said processing unit (CPU) is bidirectionally connected to said static pressure generator (PT), for making adjustable the value of static pressure $P_1$ generated and for receiving therefrom an acknowledgement signal of the occurred generation, and also to said generator (PG) for commanding the generation of the electrical signal and for receiving therefrom said electrical signal.

4. Device according to any of claims 1 to 3, characterized in that said electrical signals generated by said signal generator (PG) are electrical pulses and the signal-analyzing circuit (ACD, CM, DFT) comprises:
—an analog-to-digital converter (ADC) for converting into digital form, upon receiving a command coming from said processing unit (CPU), the electrical signal coming from said microphone transducer (T), said electrical signal having been previously amplified and filtered by an amplifier (A) and by a filter (FI);
—an averager circuit (CM) for computing the average of the digital signals coming from said analog-to-digital converter (ADC) upon receiving a command coming from said processing unit (CPU);
—a transform circuit (DFT) for computing the discrete Fourier transform of the digital signals coming from said averager circuit (CM), and supplying said processing unit (CPU) with said first, second and third sets of amplitude samples.

5. Device according to claim 2, characterized in that said electrical signals generated by said signal generator (PG) are white noise and said signal-analyzing circuit (ADC, CM, DFT) comprises:
—an analog-to-digital converter (ADC) for converting into digital form, upon receiving a command coming from said processing unit (CPU), the electrical signal coming from said microphone transducer (T), said electrical signal having been previously amplified and filtered by an amplifier (A) and by a filter (FI);
—a circuit (CM) for calculating the function of mutual correlation between the digital signals coming from said analog-to-digital converter (ADC) and the white noise which said circuit (CM) receives from said generator (PG) via said processing unit (CPU);
—a transform circuit (DFT) for computing the discrete Fourier transform of the digital signals coming from said circuit (CM), and supplying said processing unit (CPU) with said first, second and third sets of amplitude samples.

**Revendications**

1. Dispositif pour mesurer l'impédance acoustique de l'oreille obtenue au moyen de la détermination de la function de transfert d'une boucle ouverte comprenant une sonde (SE), qui peut être hermétiquement couplée à l'oreille ou à des cavités standard de calibrage respectivement et, couplés à la sonde par l'intermédiaire de petits tuyaux (R1, R2, R3), un transducteur électroacoustique (T) d'excitation, un transducteur microphonique (M) et un générateur de pressione statique (PT) qui engendre des valeurs sélectionnables de pression statique (Pi), ledit dispositif comprenant en outre:
—un générateur de signal électrique (PG) qui fournit un signal d'excitation en courant alternatif, compris dans le domaine des fréquences audibles, au transducteur d'excitation (T) qui le convertit dans un signal acounstique de test;
—un circuit analyzeur de signal (ADC, CM, DFT) alimenté par le signal provenant du transducteur microphonique (M) à l'émission dudit signal acounstique de test; et
—un dispositif de traitement (CPU) connecté de façon à recevoir le signal de sortie dudit circuit analyzeur de signal (ADC, CM, DFT) et calculant l'impedance acoustique de l'oreille sur la base dudit signal de sortie; caractérisé en ce que le générateur de signal électrique (PG) est un générateur de signaux à large spectre de fréquence qui contiennent des fréquences au moins entre 100 Hz et 4.500 Hz, en ce que le circuit analyzeur de signal (ADC, CM, DFT) fournit des échantillons d'amplitude de la répose du transducteure microphonique (M) et en ce que au dispositif de traitement (CPU) est connectée une mémoire (ME) où les échantillons d'amplitude des tests de calibrage, suivant lesquels la sonde (SE) est couplée aux cavités standard de calibrage, et les échantillons d'amplitude du test de l'oreille, suivant lequel la sonde (SE) est couplée à l'oreille même, peuvent être stockés pour des valeurs différentes de pression statique (Pi) et de fréquence, et peuvent être ultérieurement processés.

2. Dispositif selon la revendication 1, caractérisé en ce que ledit dispositif de traitement (CPU) est apte à effectuer l'opération suivante sur les échantillons d'amplitude stockés dans la mémoire (ME):

$$Z_{xi}(\omega) = \frac{\gamma P_i}{j\pi c a_1^2} \cot \frac{\omega L_1}{c} \cdot \frac{\dfrac{S_{xi}(\omega)}{S_{1i}(\omega)}\left(\dfrac{S_{1i}(\omega)}{S_{2i}(\omega)} - 1\right)}{\left(\dfrac{a_2^2 \cot \omega L_1/c}{a_1^2 \cot \omega L_2/c} - 1\right) - \dfrac{S_{xi}(\omega)}{S_{1i}(\omega)}\left(\dfrac{a_2^2 \cot \omega L_1/c}{a_1^2 \cot \omega L_2/c} - \dfrac{S_{1i}(\omega)}{S_{2i}(\omega)}\right)}$$

où:

6

$Z_{xi}(\omega)$ = impédance acoustique de l'oreille pour une fréquence angulaire $\omega$ particulière et pour une pression statique Pi;

$S_{xi}(\omega)$ = échantillons d'amplitude du test de l'oreille pour $\omega$ et Pi;

$S_{1i}(\omega)$ = échantillons d'amplitude du test de la première cavité standard de calibrage pour $\omega$ et Pi;

$S_{2i}(\omega)$ = échantillons d'amplitude du test de la seconde cavité standard de calibrage pour $\omega$ et Pi;

$a_1$, $L_1$ = rayon et longeur, respectivement, de la première cavité cylindrique standard de calibrage;

$a_2$, $L_2$ = rayon et longeur, respectivement, de la seconde cavité cylindrique standard de calibrage;

c = vitesse de propagation du son dans l'air;

$\gamma$ = paramètre préétabli.

3. Dispositif selon la revendication 2, caractérisé en ce que ledit dispositif de traitment (CPU) est connecté de façon bidirectionnelle audit générateur de pression statique (PT) pour rendre variable la valeur de pression statique (Pi) engendrée et pour en recevoir un signal de confirmation que cette génération a eu lieu, et ainsi audit générateur (PG) pour commander la génération du signal électrique et pour en recevoir ledit signal électrique.

4. Dispositif selon l'une des revendications 1 à 3, caractérisé en ce que lesdits signaux électrique engendrés par ledit générateur de signal (PG) sont des impulsions électriques et le circuit analyseur de signal (ADC, CM, DFT) comprend:

—un convertisseur analogique-numérique (ADC) qui transforme sous forme numérique, en recevant une commande provenant dudit dispositif de traitement (CPU), le signal électrique provenant du transducteur microphonique (T), ce signal électrique étant préalablement amplifié et filtré par un amplificateur (A) et un filtre (FI);

—un circuit (CM) de calcul de la moyenne des signaux numériques provenant dudit convertisseur analogique-numérique (ADC), d'après une commande qui arrive dudit dispositif de traitement (CPU);

—un circuit (DFT) qui calcule la transformée discrète de Fourier des signaux numériques provenant dudit circuit (CM) de calcul de la moyenne, et qui fournit audit dispositif de traitement le premier, deuxième et troisième groupe d'échantillons d'amplitude.

5. Dispositif selon la revendication 2, caractérisé en ce que ledit signal électrique engendré par ledit générateur (PG) est constitué par du bruit blanc et ledit circuit analyseur de signal (ADC, CM, DFT) comprend:

—un convertisseur analogique-numérique (ADC) qui transforme sous forme numérique, en recevant une commande provenant dudit dispositif de traitement (CPU), le signal électrique provenant dudit transducteur microphonique (T), ce signal électrique étant préalablement amplifié et filtré par un amplificateur (A) et un filtre (FI);

—un circuit (CM) de calcul de la fonction de corrélation mutuelle entre les signaux numériques provenant dudit convertisseur analogique-numérique (ADC) et le bruit blanc que ledit circuit (CM) reçoit dudit générateur (PG) à travers ledit dispositif de traitement (CPU);

—un circuit (DFT) qui calcule la transformée discrète de Fourier des signaux numériques provenant dudit circuit (CM) et qui fournit audit dispositif de traitement (CPU) le premier, deuxième et troisième groupe d'échantillons d'amplitude.

**Patentansprüche**

1. Vorrichtung zur Messung der akustischen Ohrimpedanz unter Auswertung der Transferfunktion einer offenen Schleife, zu der eine hermetisch mit dem Ohr bzw. mit Eich-Standardhohlräumen zu koppelnde Sonde (SE) und, über Röhrchen (R1, R2, R3) mit der Sonde gekoppelt, ein erregender elektroakustischer Umsetzer (T), ein Mikrophon-Umsetzer (M) und ein Generator statischen Drucks (PT), der wählbare Werte statischen Drucks ($P_i$) erzeugt, gehören, mit weiterhin folgenden Teilen:

—einem elektrischen Signalgenerator (PG), der ein erregendes Wechselstromsignal im Hörfrequenzbereich an den erregenden Umsetzer (T) abgibt und ihn zur Abgabe eines Test-Lautsignals anregt,

—einer signalanalysierenden Schaltung (ADC, CM, DFT), die auf die Abgabe des Test-Lautsignals hin von dem vom Mikrophon -Umsetzer (M) abgegebenen Signal gespeist wird, und

—einer Verarbeitungseinheit (CPU), die vom Ausgangssignal der signalanalysierenden Schaltung (ADC, CM, DFT) gespeist wird und hieraus die akustische Impedanz des Ohrs für die entsprechenden Daten berechnet, dadurch gekennzeichnet, daß der elektrische Signalgenerator (PG) ein Signale eines breiten Frequenzspektrums mit Frequenzen wenigstens im Bereich zwischen 100 Hz und 4500 Hz abgebender Generator ist, daß die signalanalysierende Schaltung (ADC, CM, DFT) Amplitudenabtastwerte des Antwortsignals des Mikrophon-Umsetzers (M) liefert und daß der verabeitenden Schaltung (CPU) ein Speicher (ME) zugeordnet ist, in dem die Amplitudenabtastwerte von Eichtests, bei denen die Sonde (SE) mit den Eich-Standardhohlräumen gekoppelt ist, und die Amplitudenabtastwerte des Ohrtests, bei dem die Sonde (SE) mit dem getesteten Ohr gekoppelt ist, für verschiedene statische Drücke ($P_1$) und für die verschiedenen Frequenzwerte speicherbar sind und für die weitere Verarbeitung lesbar sind.

2. Vorrichtung nach Anspruch 1, dadurch gekennzeichnet, daß die Verarbeitungseinheit (CPU) zur Durchführung der folgenden Verarbeitungsoperation der im Speicher (ME) gespeicherten Amplitudenabtastwerte aufgebaut ist:

7

$$Zxi(\omega) \approx \frac{\gamma P_i}{j\pi c a_1^2} \cot \frac{\omega L_1}{c} \cdot \frac{\dfrac{S_{xi}(\omega)}{S_{1i}(\omega)} \left( \dfrac{S_{1i}(\omega)}{S_{2i}(\omega)} - 1 \right)}{\left( \dfrac{a_2^2 \cot \omega L_1/c}{a_2^2 \cot \omega L_2/c} - 1 \right) - \dfrac{S_{xi}(\omega)}{S_{1i}(\omega)} \left( \dfrac{a_2^2 \cot \omega L_1/c}{a_1^2 \cot \omega L_2/c} - \dfrac{S_{1i}(\omega)}{S_{2i}(\omega)} \right)}$$

wobei:

$Z_{xi}(\omega)$ = akustische Ohrimpendanz für eine gegebene Winkelfrequenz $\omega$ und für den statischen Druck $P_i$;

$S_{xi}(\omega)$ = Ohr-Amplitudenabtastwerte für $\omega$ und $P_i$;

$S_{1i}(\omega)$ = Amplitudenabtastwerte des ersten Eich-Standardhohlraums für $\omega$ und $P_i$;

$S_{2i}(\omega)$ = Amplitudenabtastwerte des zweiten Eich-Standardhohlraums für $\omega$ und $P_i$;

$a_1$, $L_1$ = Radius bzw. Länge des ersten zylindrischen Eich-Standardhohlraums;

$a_2$, $L_2$ = Radius bzw. Länge des zweiten zylindrischen Eich-Standardhohlraums;

$c$ = Schallgeschwindigkeit in Luft; und

$\gamma$ = ein fester Parameter.

3. Vorrichtung nach Anspruch 2, dadurch gekennzeichnet, daß die Verarbeitungseinheit (CPU) für eine Signalübertragung in beiden Richtungen mit dem Generator des statischen Drucks (PT) zur Justierbarmachung des Werts des erzeugten statischen Drucks $P_i$ und zum Empfang eines Bestätigungssignals der durchgeführten Erzeugung vom Generator, und außerdem mit dem elektrischen Signalgenerator (PG) zum Befehlen der Erzeugung des elektrischen Signals und zum Empfangen dieses elektrischen Signals vom Generator verbunden ist.

4. Vorrichtung nach einem der Ansprüche 1 bis 3, dadurch gekennzeichnet, daß die vom elektrischen Signalgenerator (PG) erzeugten elektrischen Signale elektrische Impulse sind und die signalanalysierende Schaltung (ADC, CM, DFT) folgende Einzelschaltungen umfaßt:

—einen Analog/Digital-Umsetzer (ADC) zum Umsetzen des vom Mikrophon-Umsetzer (T) kommenden elektrischen Signals, nachdem es zunächst von einem Verstärker (A) und einem Filter (FI) verstärkt und gefiltert worden ist, in digitale Form bei Empfang eines von der Verarbeitungseinheit (CPU) kommenden Befehls;

—eine mittelwertbildende Schaltung (CM) zum Berechnen des Mittelwerts der vom Analog/Digital-Umsetzer (ADC) kommenden digitalen Signale bei Empfang eines von der Verarbeitungseinheit (CPU) kommenden Befehls;

—eine Transform-Schaltung (DFT) zum Berechnen der diskreten Furie-Transformierten der von der mittelwertbildenden Schaltung (CM) kommenden digitalen Signale und zum Abgeben der ersten, der zweiten und der dritten Gruppe von Amplitudenabtastwerten an die Verarbeitungseinheit (CPU).

5. Vorrichtung nach Anspruch 2, dadurch gekennzeichnet, daß die vom Signalgenerator (PG) erzeugten elektrischen Signale weißes Rauschen sind und die signalanalysierende Schaltung (ADC, CM, DFT) folgende Einzelschaltungen umfaßt:

—einen Analog/Digital-Umsetzer (ADC) zum Umsetzen des vom Mikrophon-Umsetzer (T) kommenden elektrischen Signals, nachdem es zunächst von einem Verstärker (A) und einem Filter (FI) verstärkt und gefiltert worden ist, in digitale Form bei Empfang eines von der Verarbeitungseinheit (CPU) kommenden Befehls;

—ein Schaltung (CM) zum Berechnen der Funktion der gegenseitigen Korrelation zwischen den vom Analog/Digital-Umsetzer (ADC) kommenden Digitalsignalen und dem weißen Rauschen, das diese Schaltung (CM) vom Signalgenerator (PG) über die Verarbeitungeinheit (CPU) empfängt;

—eine Transform-Schaltung (DFT) zum Berechnen der diskreten Furie-Transformierten der von dieser Schaltung (CM) kommenden digitalen Signale und zum Abgeben der ersten, der zweiten und der dritten Gruppe von Amplitudenabtastwerten and die Verarbeitungseinheit (CPU).

FIG .1

Fig 2

Fig. 3